# EUROPEAN PATENT APPLICATION

(11) **EP 1 580 558 A1**
(43) Date of publication of application: **28.09.2005**
(21) Application number: 03774149.3
(22) Date of filing: 21.11.2003
(51) Int. Cl.: G01N 33/573, G01N 33/569

(54) **METHOD OF INSPECTING STAPHYLOCOCCUS AUREUS**

(30) Priority: 22.11.2002 JP 2002339525
(71) Applicant: DAIICHI PURE CHEMICALS CO., LTD., Tokyo 103-0027 (JP)
(72) Inventor: HORII, Toshinobu, NAGOYA-SHI, Aichi 464-0858 (JP); KONDO, Akira, C/O DAIICHI PURE CHEMICALS CO., LTD., RYUGASAKI-SHI, Ibaraki 301-0852 (JP); KANNO, Takashi, HAMAMATSU-SHI, Shizuoka 431-3125 (JP); MAEKAWA, Masato, HAMAMATSU-SHI, Shizuoka 431-3125 (JP)
(74) Representative: Hartz, Nikolai
(86) International application number: PCT/JP2003/014912
(87) International publication number: WO 2004/048976

(57) **Abstract**

This invention relates to a method for testing *S. aureus* in a specimen wherein DNase produced by the *S. aureus* is directly detected or quantitatively determined by an immunoassay without cultivating the *Staphylococcus aureus,* and a test kit for testing *S. aureus* in a specimen by such test method.

Use the present invention enables detection of *S. aureus* in the sample in a short time and at a high sensitivity without cultivating the *S. aureus.*

## Description

### Technical Field

This invention relates to a method for directly testing *Staphylococcus aureus* in a specimen without cultivating *Staphylococcus* aureus.

### Background ART

*Staphylococcus aureus (S. aureus)* is a bacterium of *Staphylococcus spp.* which is gram-positive and which usually appears under the microscope as irregular grape-like clusters, and it is also a major pathogenic bacterium which produces various toxins and enzymes that induce purulent diseases in human and mammals. *S. aureus* is also a typical bacterial strainwhich causes food poisoning in human.

A popular procedure that has often been used in detecting *S. aureus* is a procedure wherein pure cultivation is conducted ina selective isolation medium, and suspicious colonies are chosen for further identification tests of the bacterial species, and the identification has been conducted by using coagulase produced by *S. aureus.* Coagulase is an enzyme which coagulates human or rabbit plasma, and *S*. *aureus* can be differentiated from coagulase-negative staphylococci (CNS) by checking the coagulase production.

In addition, DNase test is also used for differentiation of *S*. *aureus* from CNS since DNase is produced by *S. aureus* but not by CNS next to coagulase. In this DNase test, *S. aureus* is identified by using the phenomenon that, when a streaked DNAplate culture is cultivated overnight and to which 1.5N hydrochloric acid is added, white clouding occurs in the part where DNA is present while such whitening is not observed near the bacterial plaque where DNA decomposition by the DNase has taken place.

The conventional test methods, however, all required cultivation of the bacteria, and a substantial time was needed before the identification of the bacterium. When the results of the identification were obtained, patients were often too serious to utilize the test results. In addition, there has been a fair possibility that the bacterium present in a minute amount in blood is left undetected in patients receiving a large amount of antibacterial. Furthermore, the former method was associated with the risk that bacteria other than *Staphylococcus spp.* may grow under the same cultivation conditions.

A method has also been developed in which detection time has been reduced and specific detection of *S*. *aureus* has been enabled. In this method, precultivation is conducted and the DNase in the culture supernatant is measured by an enzyme immunoassay for identification of the bacterium (Brakstad et al . , APIMS 97, pages 166-174 (1989); Brakstad et al . , APIMS 103, pages 209-218 (1995)) . Although pure culture is not required in this method for identification of the bacterium, a precultivation is still required to improve the detection sensitivity (detection limit, 0.4 ng/mL or higher). An enzyme immunoassay by fluorescence method has also been developed (Meyrand et al., Lett Appl Microbiol 29, pages 216-220 (1999)). The detection limit of this method, however, is 1 ng/mL.

Another attempt that has been made for quick, high sensitivity detection of bacterial infection is gene amplification (Kato, I., Protein, Nucleic Acid and Enzyme, Vol. 35, page 2957 (1990)) . Since *S*. *aureus* is an indigenous bacterium, its detection is associated with the risk shared by indigenous bacteria that those not infected are also detected as positive by the minute contamination of the bacterial gene.

In the meanwhile, MRSA (methicillin-resistant *S*. *aureus)* has attracted special attention among *S*. *aureus* since it is a causative bacterium of nosocomial infection, and a convenient and fast identification method is important. MRSA has been traditionally identified by evaluating chemical sensitivity in a test cultivation of the bacteria separated from a clinical material. However, a method utilizing detection of *mecA* which is a gene involved in the antibiotic resistance of MRSA has been recently developed, and this method has enabled the MRSA identification with no need to wait the results of the sensitivity test by utilizing gene amplification (Ubukata et al., J. Clin. Microbiol., 30, pages 1728-1733 (1992)) . This method is already in clinical use.

This *mecA* gene, however, is sometimes found in *Staphylococcus* other than *S*. *aureus* such as CNS, and detection of *mecA* gene has been associated with the difficulty of clearly differentiating between MRSA and CNS.

### Disclosure of the Invention

An object of the present invention is to provide a method which is capable of detecting *S*. *aureus* in a specimen in a short time and at a high sensitivity without cultivating *S*. *aureus.*

In view of the situation as described above, the inventors of the present invention have made an intensive investigation on quick tests for detecting *S. aureus* in a specimen, and found that an immunoassay of high sensitivity and high precision using an antibody against DNase specifically produced by *S*. *aureus* is capable of directly detecting or quantitatively determining the DNase antigen in the blood, urine, sputum, spinal fluid, pleural effusion, ascites, pus, or other body fluid components from patients infected by *S. aureus* without any need for cultivating the bacterium, and that *S*. *aureus* in a specimen can be tested in a short time by using such method. The present invention has been completed on such a finding.

Accordingly, the present invention provides a method for testing *S. aureus* in a specimen wherein DNase produced by the *S. aureus* is directly detected or quantitatively determined by an immunoassay without cultivating *S*. *aureus.*

The present invention also provides a test kit for testing *S. aureus* in a specimen by the test method as described above wherein the kit at least comprises an antibody for the DNase and a reagent for detecting the labeled DNase.

The present invention has enabled to test *S*. *aureus* in a body fluid component without any need for cultivating the *S*. *aureus,* and to conveniently and quickly test whether or not the patient is infected by *S*. *aureus.* The present invention can also determine the infection by *S*. *aureus* including MRSA.

### Brief Description of the Drawings

FIG. 1 is a view showing the standard curve of purified DNase measured by sandwich enzyme immunoassay 1.
FIG. 2 is a view showing the standard curve of the purified DNase measured by sandwich enzyme immunoassay 2.
FIG. 3 is a view showing the standard curve of the purified DNase measured by sandwich enzyme immunoassay 3.
FIG. 4 is a view showing amount of the DNase in the supernatant that has been measured over time.
FIG. 5 is a view showing measurements of the DNase in serum of healthy donors (3 specimens), and serum (8 specimens) and urine (4 specimens) of the patients suffering from *S*. *aureus.*

### Best Mode for Carrying Out the Invention

The method for testing *S. aureus* of the present invention is a method wherein DNase produced by *S.* aureus is directly detected in the specimen by an immunoassay.

Accordingly, the present invention does not require precultivation of the specimen (test specimen) or increase in the concentration of the DNase in the specimen by centrifugation, column separation, precipitation, or the like.

In the present invention, the term "*S*. aureus" includs methicillin-resistant *S*. *aureus*.

The specimen used in the present invention is not limited as long as there is possibility that *S*. aureus is present, and exemplary specimens include blood, urine, sputum, spinal fluid, pleural effusion, ascites, pus, and other body fluid components. More specifically, the present invention is capable of directly detecting or quantitatively determining the DNase without precultivation of the test specimen or increase in the concentration of the DNase of the test specimen not only when the specimen are those containing the bacteria at a high concentration such as sputum or urine but also when the specimen are those containing the bacteria at a low concentration (for example, 10 to 10³ cfu/mL) such as blood, pleural effusion, or ascites.

In addition to the use of the specimen with no further dilution, the specimen may also be used after diluting with a buffer such as phosphate buffer. The buffer used in the dilution of the specimen may simultaneously contain an adequate protein such as BSA, HSA, gelatin, or the like for stabilization of the antigen.

The immunoassay used in the present invention is not particularly limited as long as it is an immunoassay of high sensitivity which can measure the DNase produced by *S*. *aureus* to the order of 100 pg/mL or less, and more preferably 10 pg/mL or less, and exemplary immunoassays include enzyme immunoassay, radioimmmunoassay, immunochromatography, fluorescent immunoassay, and other immunoassays known in the art. The preferred, however, is an enzyme immunoassay in view of the safety and the sensitivity.

An enzyme immunoassay is an immunoassay wherein amount of the relevant antibody or antigen is measured by detecting enzyme activity of the enzyme labeled antibody using an enzyme such as peroxidase, alkaline phosphatase, or β-galactosidase. Enzyme immunoassays using various enzyme detection means are known such as colorimetric enzyme immunoassay, fluorescent enzyme immunoassay, and chemiluminescent enzyme immunoassay (CLEIA). In the colorimetric enzyme immunoassay, enzyme activity is quantitatively determined by measuring coloring of the chromogenic substance generated by decomposition of the enzymatic substrate such as hydrogen peroxide/o-phenylenediamine, p-nitrophenylphosphate,or o-nitrophenyl-β-D-galactopyranoside by the enzyme. In the fluorescent enzyme immunoassay, fluorescence is generated by decomposition of the fluorescent substrate such as 4-hydroxyphenyl acetic acid, 4-methyl-umbelliferyl phosphate, or 4-methyl-umbelliferyl-β-galactoside by the catalytic activity of the enzyme, and the fluorescence intensity is measured to quantitatively determine the enzyme activity. In the chemiluminescent enzyme immunoassay (CLEIA), chemiluminescent substance is excited by the catalytic activity of the enzyme, and amount of the light emitted when it returns to the ground state is measured to quantitatively determine the enzyme activity. Among these, use of a chemiluminescent enzyme immunoassay is particularly preferable in the present invention since no light source is required for the chemiluminescence of the chemiluminescent substance, and detection at a sensitivity higher than the colorimetric or the fluorescent immunoassays is possible.

Exemplary enzymes used in the CLEIA include peroxidase, alkaline phosphatase, β-galactosidase, glucose oxidase, and glucose-6-phosphodehydrogenase, and exemplary chemiluminescent substances include luminols such as luminol derivatives and isoluminol derivatives, lucigenin(N,N'-dimethyl-9,9'-bisacridinium nitrate), and bis(2,4,6-trichlorophenyl)oxalate. Among these, the preferred is the combination of luminol /hydrogen peroxide for the substrate with peroxidase (Tsuji, A., Protein, Nucleic Acid and Enzyme, Special Edition, vol. 31, pages 51-63 (1987)). If necessary, D-(-)-luciferin, 6-hydroxybenzothiazole, p-iodophenol, or the like may be simultaneously incorporated in the chemiluminescent system to enhance the chemiluminescence.

The immunoassay of the present invention is not particularly limited for its measurement procedure, and the assay may be conducted either by a competitive assay or a sandwich assay. However, use of a sandwich assay is preferable in view of conducing the assay at a high sensitivity.

Next, an embodiment of the test method for *S*. *aureus* using sandwich assay is described in detail.
1) Antibody against DNase produced by *S*. *aureus* is immobilized on an adequate solid carrier by adsorption.
2) The antibody is brought in contact with the specimen containing the *S. aureus* for specific bonding of the DNase produced by the *S. aureus* in the specimen with the anti-DNase antibody immobilized on the solid carrier to thereby immobilize the DNase in the specimen to the solid carrier.
3) The carrier having DNase immobilized thereon is brought in contact with a solution containing a labeled antibody against the DNase (secondary antibody) to bind the secondary antibody to the solid carrier through the immobilized anti-DNase antibody and the DNase.
4) The thus immobilized secondary antibody is measured by means of the label of the secondary antibody to detect or quantitatively determine the DNase in the specimen.

The antibody against the DNase used in the test method of the present invention may be either a polyclonal antibody or a monoclonal antibody, and this antibody can be produced by the method known in the art using the purified antigen.

The secondary antibody may comprise an antibody in its entirety. However, in view of reducing the nonspecific reactions to the lowest possible level, the secondary antibody may preferably comprise a fragment of the antibody such as Fab, Fab' , or F(ab')2 produced by treating the antibody with an enzyme such as pepsin or by reduction treatment. The combination of the primary antibody and the secondary antibody may be a combination of a monoclonal antibody or a fragment thereof recognizing different epitopes, or a combination of a polyclonal antibody and a monoclonal antibody or a fragment thereof.

Non-limiting Examples of the solid carrier used for immobilizing the primary antibody include glass or plastic tube, plate, well, or beads, magnetic particles, latex, membrane, and fibers.

The immobilization can be accomplished by physical adsorption, or alternatively, by chemical bonding using a crosslinking agent such as glutaraldehyde or carbodiimide. For example, immobilization may be conducted by contacting the antibody diluted with phosphate buffer with the surface of the solid carrier, and incubating the carrier at 37°C for 60 minutes.

The labeling of the secondary antibody may be accomplished by direct labeling with a radioisotope, an enzyme, biotin, a fluorescent substance, a chemiluminescent substance, gold colloid, latex, ferrite particle, or the like. The secondary antibody, however, is preferably labeled with an enzyme as described above in view of high safety and expectation for good measurement results. Exemplary preferable enzymes used for the labeling include peroxidase, alkaline phosphatase, and galactosidase which are highly stable and whose enzyme activity can be readily determined.

The secondary antibody bonded can be detected or quantitatively determined by any method known in the art, for example, by directly detecting or quantitatively determining the secondary antibody itself that has been labeled with an enzyme, a luminescent substance, a fluorescent substance, or the like, or by using a tertiary antibody which specifically binds to the secondary antibody and labeling this tertiary antibody by various methods and detecting or quantitatively determining the label of the tertiary antibody.

The secondary antibody is preferably detected by reacting the secondary antibody labeled with an enzyme with a substrate (chromogenic substrate, fluorescent substrate, or chemiluminescent substrate) which is specific for the enzyme, and detecting the signal associated with the color development, fluorescence, luminescence, or the like induced by the reaction with a measuring device. Use of the chemiluminescent substrate which enables high sensitivity detection is particularly preferable.

The test kit for infections caused by *S*. *aureus* of the present invention is a kit for carrying out the test method for *S. aureus* in the specimen as described above, and the kit at least comprises an antibody against the DNase as described above (two antibodies when the immunoassay used is a sandwich assay), and a reagent for detecting the label such as an enzyme, and optionally, a buffer solution for diluting the specimen, a buffer solution for diluting various reagents, a washing solution, and the like.

### Examples

Next, the present invention is described in further detail by referring to the Examples which by no means limit the scope of the invention.

### Example 1

### (1) Preparation of a plate having primary antibody immobilized thereto

Rabbit or sheep anti-DNase polyclonal antibody diluted with 25 mM phosphate buffer solution (PBS) containing 150 mM NaCl to 2 µg/mL was dispensed in the wells of a plate at 50 µL/well, and the plate was allowed to stand overnight at 4°C for immobilization of the antibody. The content was removed from the plate the next day, and after adding 280 µL/well of PBS containing 0.2% bovine serum albumin (BSA) (0.2% BSA-PBS), the plate was allowed to stand overnight or longer at 4°C. In use, the plate washed three times with PBS containing 0.05% Tween20 (PBS-T) was used for the reaction.

### (2) Preparation of biotinylated secondary antibody

500 µL (1 mg/mL) of sheep anti-DNase polyclonal antibody was dialyzed against 0.1M sodium hydrogen carbonate. A solution of 0.05 mg of Sulfo-NHS-LC-Biotin (manufactured by Pierce) in 50 µL of the carbonate buffer solution was added dropwise to the antibody solution with stirring. After stirring at room temperature for 4 hours, and dialyzing against PBS overnight at 4°C, the antibody solution was used as the biotinylated secondary antibody.

### Example 2

### (1) Measurement of DNase by sandwich enzyme immunoassay (1)

50 µL/well of purified DNase (manufactured by Toxin Technology) diluted with 0.2% BSA-PBS to 0. 1, 1, 10, and 100 ng/mL was added to the wells of the microtiter plate having the sheep anti-DNase polyclonal antibody immobilized thereto produced in Example 1(1). After allowing the reaction to take place at room temperature for 1 hour, the wells were washed with PBS-T, and 50 µL/well of rabbit anti-DNase polyclonal antibody diluted with 0.2% BSA-PBS to 2 µg/mL was added as the secondary antibody. The reaction was then allowed to proceed at room temperature for 1 hour, and the wells were washed with PBS-T. In the meanwhile, HRP-labeled pig anti-rabbit IgG antibody (manufactured by DAKO) was diluted with 0.2% BSA-PBS to 4000 folds, and 50 µL/well of this dilution was added to the well, and the reaction was allowed to proceed at room temperature for 1 hour. After washing, 50 µL/well of 0.1M citrate buffer solution containing 2 mg/mL o-phenylenediamine and 9. 5 mM hydrogen peroxide, pH 5 was added, and the reaction was allowed to proceed at room temperature for 30 minutes, and the reaction was ceased by adding 50 µL/well of 1.5N sulfuric acid. The measurement was conducted by using a plate reader for ELISA at a wavelength of 492 nm.

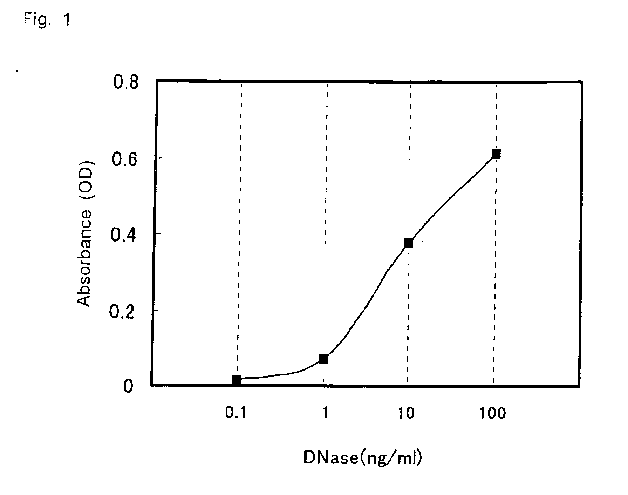

The resulting standard curve is shown in FIG. 1. The measurement was possible in the DNase concentration range of 0.1 to 100 ng/mL.

### (2) Measurement of DNase by sandwich enzyme immunoassay (2)

50 µL/well of purified DNase diluted with 0.2% BSA-PBS to 0.1, 1, 10, 100, and 1000 ng/mL was added to the wells of the microtiter plate having the rabbit anti-DNase polyclonal antibody immobilized thereto produced in Example 1(1). After allowing the reaction to take place at room temperature for 1 hour, the wells were washed with PBS-T, and 50 µL/well of biotinylated sheep anti-DNase polyclonal antibody diluted with 0.2% BSA-PBS to 3 µg/mL produced in Example 1 (2) was added as the secondary antibody. The reaction was then allowed to proceed at room temperature for 1 hour, and the wells were washed with PBS-T. In the meanwhile, avidinylated HRP (manufactured by Zymed) was diluted with 0.2% BSA-PBS to 4000 folds, and 50 µL/well of this dilution was added to the well, and the reaction was allowed to proceed at room temperature for 1 hour. After washing, 50 µL/well of 0. 1M citrate buffer solution, containing 2 mg/mL o-phenylenediamine and 5.9 mM hydrogen peroxide, pH 5 was added, and the reaction was allowed to proceed at room temperature for 30 minutes, and the reaction was ceased by adding 50 µL/well of 1.5N sulfuric acid. The measurement was conducted by using a plate reader for ELISA at a wavelength of 492 nm.

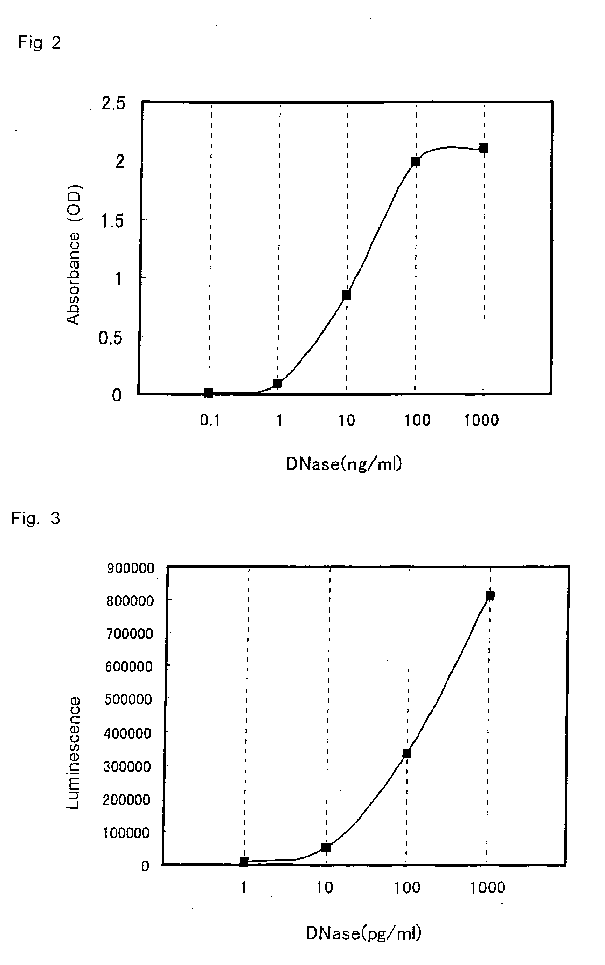

The resulting standard curve is shown in FIG. 2. The measurement was possible in the DNase concentration range of 0.1 to 100 ng/mL.

### (3) Measurement of DNase by sandwich enzyme immunoassay (3)

50 µL/well of purified DNase diluted with 0.2% BSA-PBS to 1, 10, 100, and 1000 pg/mL was added to the wells of the white microtiter plate (manufactured by Dynex technologies) having the rabbit anti-DNase polyclonal antibody immobilized thereto. After allowing the reaction to take place at room temperature for 1 hour, the wells were washed with PBS-T, and 50 µL/well of biotinylated sheep anti-DNase polyclonal antibody diluted with 0.2% BSA-PBS to 3 µg/mL produced in Example 1(2) was added as the secondary antibody. The reaction was then allowed to proceed at room temperature for 1 hour, and the wells were washed with PBS-T. In the meanwhile, avidinylated HRP was diluted with 0.2% BSA-PBS to 4000 folds, and 50 µL/well of this dilution was added to the well, and the reaction was allowed to proceed at room temperature for 1 hour. Afterwashing, 100µL/well of Super Signal ELISA Femto Substrate (manufactured by Pierce) was added, and luminescence of 10 seconds was integrated with a luminometer.

The resulting standard curve is shown in FIG. 3. The measurement was possible in the DNase concentration range of 0.1 to 1000 pg/mL.

### Example 3: Measurement of the amount of DNase in the culture supernatant over time

To 10 mL of heart-infusion broth was added 80 µL of overnight culture of the standard strain of *S*. *aureus* (NCTC8325), and the cultivation was continued. 1 mL portion of the culture was collected over time, and the collected culture was centrifuged for 2 minutes. The supernatant was filtered with 0.2 µm membrane, and the filtrate was stored at -30°C until measurement. In order to measure the amount of the DNase secreted in accordance with the growth of the bacterium, the sample was diluted 100 to 5000 folds with 0.2% BSA-PBS for measurement by ELISA.

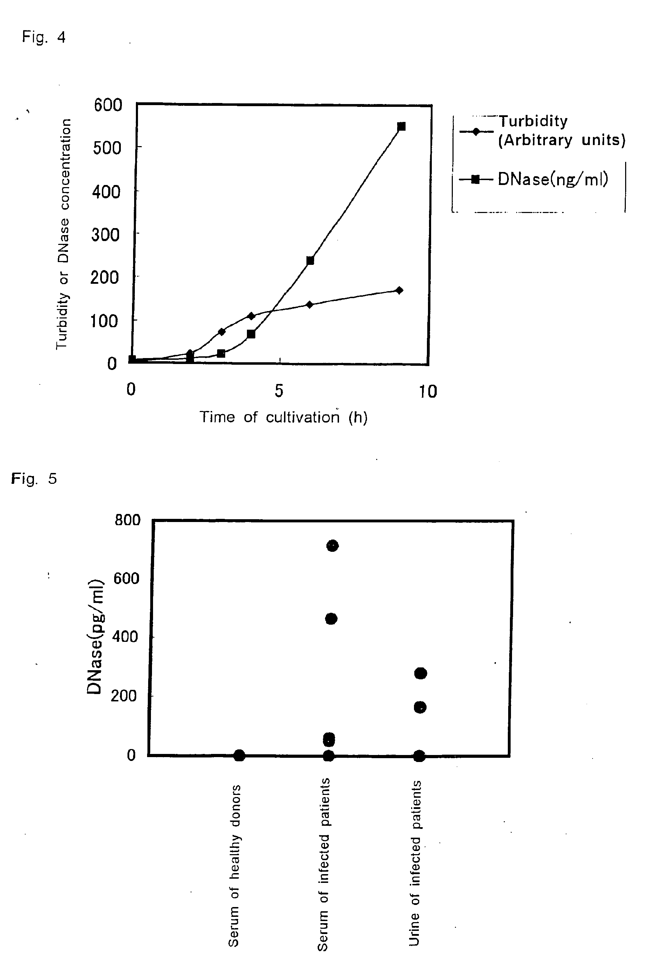

The results are shown in FIG. 4. As demonstrated by the results, production of DNase increased corresponding to the growth phase of the bacterium.

### Example 4: Measurement of DNase in the serum and urine of the patients

DNase in the serum and urine of the patients suffering from *S. aureus* and the healthy donors were measured according to the procedure described in Example 2 (3) . The results are shown in FIG. 5. While no DNase was detected in the serum of the healthy donor, presence of DNase was confirmed in the serum and the urine of the infected patients.

## Claims

1. A method for testing *Staphylococcus aureus* in a specimen wherein DNase produced by the *Staphylococcus aureus* is directly detected or quantitatively determined by an immunoassay without cultivating the *Staphylococcus aureus*.

2. The test method according to claim 1 wherein the immunoassay is an enzyme immunoassay.

3. The test method according to claim 1 wherein the immunoassay is an enzyme chemiluminescent immunoassay.

4. The test method according to claim 3 wherein the enzyme chemiluminescent immunoassay is the one using peroxidase for the enzyme to be labeled and a luminol for the chemiluminescent substance.

5. The test method according to any one of claims 1 to 4 wherein the specimen is a body fluid component.

6. The test method according to any one of claims 1 to 5 wherein *Staphylococcus aureus* is methicillin-resistant *Staphylococcus aureus.*

7. A test kit for testing *Staphylococcus aureus* in a specimen by the test method according to any one of claims 1 to 6 wherein the kit at least comprises an antibody for the DNase and a reagent for detecting the labeled DNase.
